# EUROPÄISCHE PATENTSCHRIFT

(11) **EP 1 475 056 B1**
(45) Veröffentlichungstag und Bekanntmachung des Hinweises auf die Patenterteilung: **13.10.2010**
(21) Anmeldenummer: 04007368.6
(22) Anmeldetag: 26.03.2004
(51) Int. Cl.: A61F 2/18, A61F 5/08

(54) **Implantat zur Spreizung der Nasenflügel**
Implant for spreading the wings of the nose
Implant pour écarter les ailes du nez

(30) Priorität: 07.05.2003 DE 20307058 U
(43) Veröffentlichungstag der Anmeldung: 10.11.2004
(73) Patentinhaber: Heinz Kurz GmbH Medizintechnik, 72144 Dusslingen (DE)
(72) Erfinder: Brusis, Tilman, Prof. Dr. med., 50931 Köln (DE); à Wengen, Daniel, Dr. med., CH-4102 Binningen (CH)
(74) Vertreter: Kohler Schmid Möbus

(56) Entgegenhaltungen:
- US-A- 5 133 754
- US-A- 5 716 405
- US-A1- 2002 173 848
- US-B1- 6 322 590

## Beschreibung

Die Erfindung betrifft ein dachförmiges Implantat zur Spreizung der Nasenflügel, das am Dreiecksknorpel befestigbar ist und aus einem in die Dachform gebogenen ebenen Streifen aus Metall gefertigt ist.

Ein solches Implantat zur Spreizung der Nasenflügel ist beispielsweise durch die US 6,322,590 B1 bekannt geworden.

Die Einpflanzung eines Implantats, d. h. eines in der Regel körperfremden Gewebsstücks oder Stoffes, in den menschlichen Körper ist in der Medizintechnik ein bereits bekanntes Verfahren, das in vielen Variationen zur Behebung funktioneller Störungen verschiedener Körperteile und/oder psychischer Beeinträchtigungen vorgenommen wird.

Eine Spreizung der Nasenflügel kann beispielsweise bei einer Verengung der Nasenklappe oder bei einem Kollaps der Weichteile des Nasenflügels indiziert sein.

Eine einfache und bekannte Methode zur Spreizung der Nasenflügel besteht darin, ein Nasenstützpflaster zu verwenden, das insbesondere auch Hochleistungssportler zur Verbesserung der Atmung einsetzen. Dieses Vorgehen ist jedoch zur dauerhaften Anwendung ungeeignet. Bei regelmäßiger Verwendung der Nasenpflaster können durch den Klebstoff Hautprobleme entstehen. Außerdem beeinträchtigt ein Nasenpflaster das äußere Erscheinungsbild eines Menschen.

Zur dauerhaften Spreizung der Nasenflügel sind daher auch operative Methoden bekannt, bei denen zur Stabilisierung der seitlichen Nasenweichteile Knorpel eingesetzt wird. Die Ergebnisse sind jedoch optisch und funktional nicht immer befriedigend. Außerdem bringt dieses Vorgehen noch andere Nachteile mit sich. Da vorzugsweise körpereigener Knorpel zur Stabilisierung verwendet wird, muss die Entnahme des Knorpels, vorzugsweise aus dem Ohr oder aus der Nasenscheidewand des Patienten, diesem Schritt vorangehen, was einen zeitlichen Aufwand und ein zusätzliches Risiko für den Patienten bedeutet. Oft zeigt sich, dass das eingesetzte Teil zu wenig Eigenspannung besitzt, um den kaudalen Luftraum der Nase in einer befriedigenden Weise offen zu halten. Das Verfahren kann sich sogar als kontraproduktiv erweisen, weil die eingesetzten Teile die Weichteile nach innen drücken und zu einer weiteren Verengung der Nase führen können.

Vorrichtungen und Verfahren zur Herstellung derartiger Transplantate oder Implantate aus weichen, knorpeligen körpereigenen Materialien oder einem entsprechenden Kunststoff mittels Schablonen mit schmetterlingsartiger Peripherie sind in der US-A-5,716,405 beschrieben. Diese sollen dann während der Operation in Gewebetaschen in der Nase eingesteckt werden, wo sie sich der Form der Nase lokal anpassen.

Auch metallische Implantate werden zur Spreizung der Nasenflügel eingesetzt. Dazu wird operativ eine Öffnung im Bereich des Flügelrandes der Nase eingebracht, durch welche das Implantat eingesetzt und am Dreiecksknorpel befestigt wird. Metallische Implantate sind wesentlich fester und elastischer als Knorpel und besitzen genügend Eigenspannung, um den nasalen Luftraum in einer dauerhaften und befriedigenden Weise offen zu halten.

Aus der US 6,322,590 B1 ist ein dachförmiges Implantat bekannt, das aus einem geraden ebenen Metallstreifen gefertigt ist. Dieser Metallstreifen weist an seinen beiden Enden Perforationen in Form von runden Löchern auf und wird in die V-förmige Dachform gebogen.

Der Erfindung liegt die Aufgabe zugrunde, bei einem Implantat der eingangs genannten Art eine erheblich höhere mechanische Stabilität zu erzielen sowie ergonomisch günstigere Implantierungsanordnungen in der Nase zu ermöglichen.

Diese Aufgabe wird durch die technischen Merkmale aus Anspruch 1 gelöst.

Das erfindungsgemäße Implantat hat den Vorteil, dass die Streifenenden gegenüber der bekannten V-förmigen Dachform im gebogenen Zustand räumlich schräg verlaufen. Diese Geometrie ermöglicht andere bzw. verbesserte Anordnungen des Implantats in der Nase. Insbesondere ist das erfindungsgemäße Implantat durch seine besondere Geometrie anatomisch und ergonomisch günstiger gestaltet, weil die Seitenflügel des Implantats nach hinten abgebogen sind und z. B. beim Schnäuzen der Nase nicht stören. Außerdem besitzt das erfindungsgemäße Implantat aufgrund der bereits im flachen Zustand des ebenen Streifens vorgesehenen Abwinklung der beiden Schenkel ein wesentlich größeres Flächenträgheitsmoment, was ihm eine erheblich höhere Stabilität verleiht.

Für die Form des Implantats gibt es mehrere Möglichkeiten. In einer einfachen nicht beanspruchten Ausführung kann das Implantat ein V-förmig gebogener Streifen sein, wobei zweckmäßigerweise die Spitze des V abgerundet ist. Das Implantat kann auch aus trapezförmig angeordneten Streifen bestehen und/oder kompliziertere Strukturen mit Verzweigungen aufweisen, die zu einer Stabilisierung beispielsweise des Nasenrückens dienen können.

Als Material für chirurgische und orthopädische Implantate sind Titan oder Titanverbindungen prädestiniert, weil sie neben einer sehr guten Biokompatibilität hohe Dauerfestigkeit und Elastizität aufweisen. Trotz einer relativ geringen Dichte im Vergleich beispielsweise zu Stahl besitzen Implantate aus solchen Materialien ausgezeichnete mechanische Eigenschaften mit einer langen Lebensdauer. Insbesondere ist die spezielle Ni-Ti-Legierung Nitinol wegen ihres Gedächtniseffekts hervorzuheben. Das erfindungsgemäße Implantat kann daher vorzugsweise aus Titan, einer Titanlegierung oder Nitinol gefertigt sein.

In einer vorteilhaften Ausgestaltung kann das Implantat Perforationen aufweisen. Damit wird einerseits das Gewicht des Implantats verringert, andererseits der Anteil von körperfremdem Material, das durch das Implantat in den menschlichen Körper eingebracht wird, so weit wie möglich reduziert. Außerdem fördern die Perforationen das Verwachsen des Implantats mit dem Gewebe. Die Perforationen sind vorzugsweise an den Enden und an dem dazwischen liegenden Mittelteil des Streifens vorgesehen und zum Beispiel als kreisrunde Löcher oder als Langlöcher ausgebildet.

Neben der guten Biokompatibilität des Materials selbst kann das Implantat auch einen körperverträglichen Überzug aufweisen.

Zur Erzielung einer fein abgestimmten Form des Implantats kann es zweckmäßigerweise mittels Lasertechnik hergestellt sein.

Nachfolgend werden bevorzugte Ausführungsbeispiele erfindungsgemäßer Implantate anhand der Zeichnung näher erläutert.

Es zeigen:
- Fig. 1: eine perspektivische Ansicht einer ersten Ausführungs- form eines erfindungsgemäßen Implantats;
- Fig. 2: eine schematische Ansicht des Implantats aus Fig. 1 in situ;
- Fig. 3: eine perspektivische Ansicht einer zweiten Ausführungsform eines erfindungsgemäßen Implantats; und
- Fig. 4: den ebenen Zuschnitt des in Fig. 3 gezeigten Implantats.

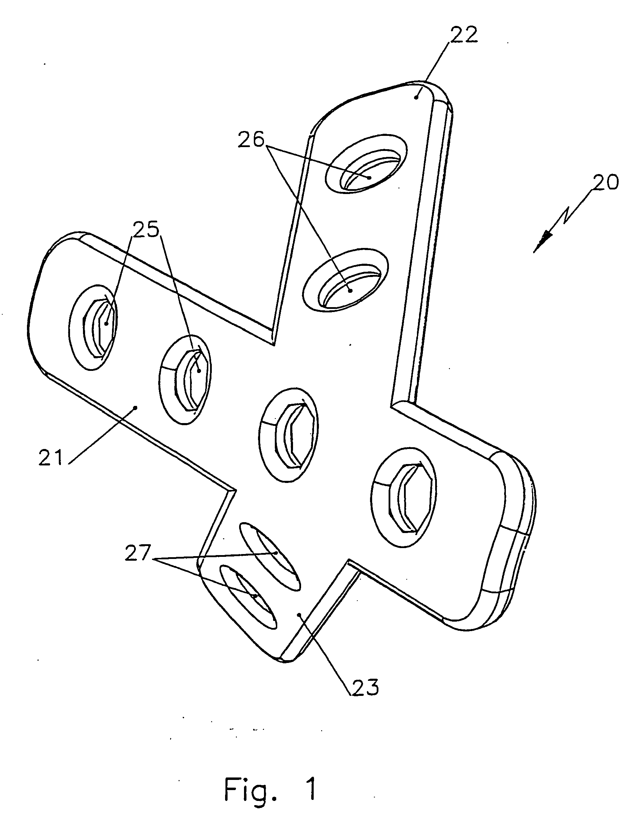

Fig. 1 zeigt eine perspektivische Ansicht einer ersten Ausführungsform eines erfindungsgemäßen Implantats 20, das aus einem zentralen Streifen 21 und zwei sich daraus symmetrisch verzweigenden Streifen 22 und 23 besteht, welche zusammen für das Implantat 20 eine dachförmige bzw. trapezförmige Kontur ergeben. Die Streifen 21, 22 und 23 weisen eine Reihe von regelmäßigen Perforationen 25, 26 und 27 auf, welche helfen, das Gewicht des Implantats 20 zu verringern, den Anteil des körperfremden Materials im Körper eines Patienten möglichst gering zu halten und das Verwachsen des Implantats 20 mit dem Gewebe zu fördern.

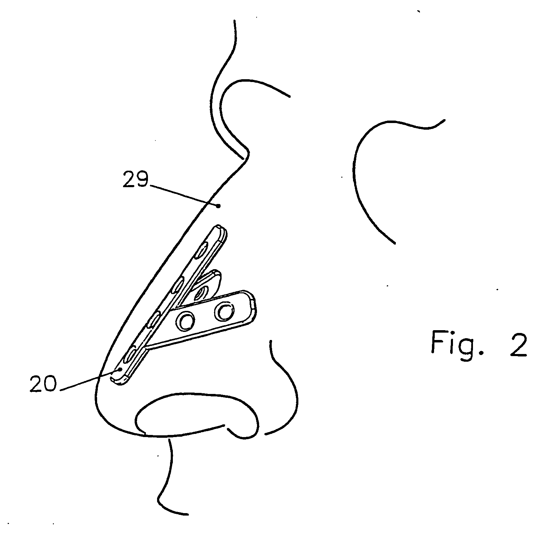

Fig. 2 zeigt schematisch die Lage des in eine Nase 29 operativ eingesetzten Implantats 20 aus Fig. 1. Es ist auch ersichtlich, wie der zentrale Streifen 21 bei diesem Implantat auch zur Gestaltung und/oder Stabilisierung des Nasenrückens dienen kann.

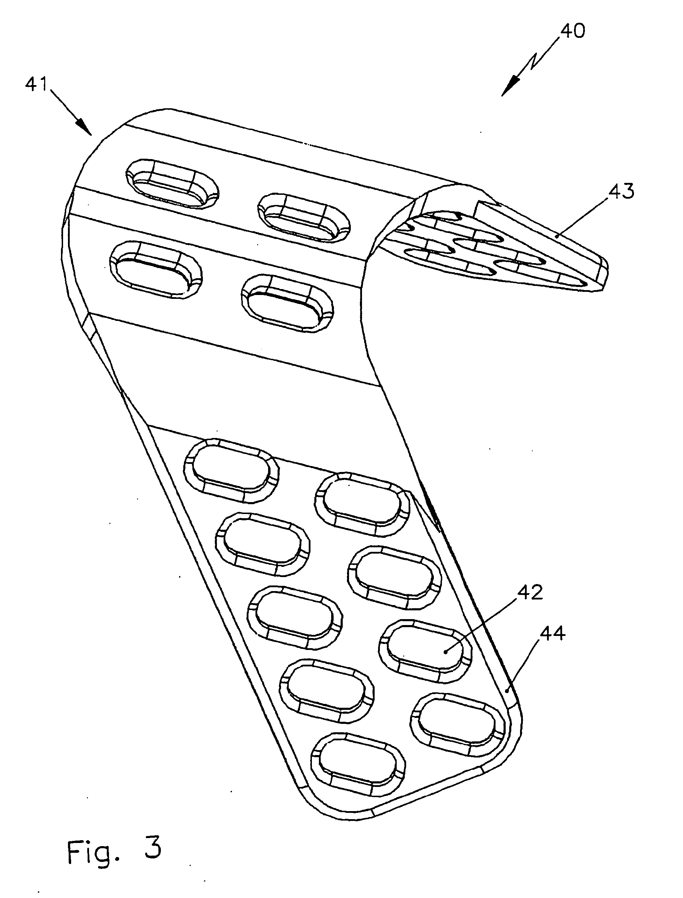

Fig. 3 zeigt in perspektivischer Ansicht eine zweite Ausführungsform eines erfindungsgemäßen Implantats 40, das aus einem in die Dachform gebogenen ebenen Streifen mit doppelreihig ausgebildeten Perforationen 42 gefertigt ist.

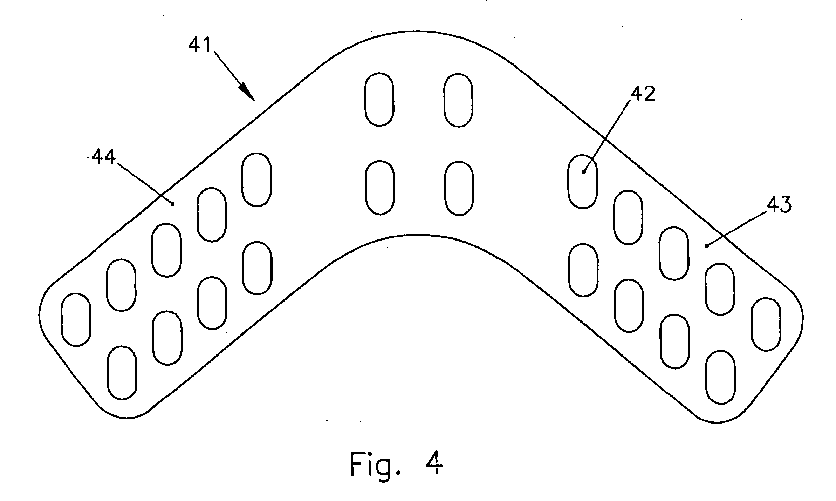

Wie in Fig. 4 gezeigt, weist der ebene Zuschnitt 41 eine V-förmige Kontur mit abgewinkelten Enden 43, 44 auf. Nach Biegen des Zuschnitts 41 in die Dachform sind die Enden 43, 44 des Streifens 41 räumlich abgeschrägt.

## Patentansprüche

1. Dachförmiges Implantat (40) zur Spreizung der Nasenflügel, das am Dreiecksknorpel befestigbar ist und aus einem ebenen Zuschnitt (41) aus Metallgefertigt ist, der in die Dachform gebogen ist,
**dadurch gekennzeichnet,**
**dass** der ebene Zuschnitt (41) bereits vor dem Biegen in die Dachform eine abgewinkelte Kontur mit zwei aus einem zentralen Mittelteil symmetrisch zu beiden Seiten sich daraus verzweigenden Seitenstreifen (43, 44) aufweist.

2. Implantat, insbesondere nach Anspruch 1, **dadurch gekennzeichnet, dass** es aus Titan, einer Titanlegierung oder Nitinol gefertigt ist.

3. Implantat nach einem der Ansprüche 1 oder 2, **dadurch gekennzeichnet, dass** es Perforationen (42) aufweist.

4. Implantat nach Anspruch 3, **dadurch gekennzeichnet, dass** die Perforationen (42) an den Seitenstreifen (43, 44) und an dem dazwischen liegenden Mittelteil des Zuschnitts (41) vorgesehen sind.

5. Implantat nach Anspruch 3 oder 4, **dadurch gekennzeichnet, dass** die Perforationen (42) als kreisrunde Löcher oder als Langlöcher ausgebildet sind.

6. Implantat nach einem der Ansprüche 1 bis 5, **dadurch gekennzeichnet, dass** es einen körperverträglichen Überzug aufweist.

7. Implantat nach einem der Ansprüche 1 bis 6, **dadurch gekennzeichnet, dass** es mittels Lasertechnik hergestellt ist.

## Claims

1. Roof-shaped implant (40) for spreading the wings of the nose, which can be fastened to the triangular cartilage and is produced from a flat metal blank (41) which is bent to form the roof shape,
**characterised in that**
the flat blank (41) already has an angled contour prior to bending and comprises two lateral strips (43, 44) branching from a central part symmetrically with respect to both sides thereof.

2. Implant, in particular according to Claim 1, **characterised in that** it is produced from titanium, a titanium alloy or Nitinol.

3. Implant according to either of Claims 1 and 2, **characterised in that** it has perforations (42).

4. Implant according to Claim 3, **characterised in that** the perforations (42) are provided in the lateral strips (43, 44) and in the central part of the blank (41) located between them.

5. Implant according to Claim 3 or 4, **characterised in that** the perforations (42) are in the form of circular holes or slots.

6. Implant according to any one of Claims 1 to 5, **characterised in that** it has a biocompatible coating.

7. Implant according to any one of Claims 1 to 6, **characterised in that** it is produced using laser technology.

## Revendications

1. Implant (40) en forme de toit pour écarter les ailes du nez, qui peut être fixé sur le cartilage triangulaire et est formé d'une découpe plane (41) en métal, qui est recourbée en forme de toit, **caractérisé en ce que** la découpe plane (41) présente déjà, avant la courbure en forme de toit, un contour coudé avec deux bandes latérales (43, 44) se ramifiant depuis une partie centrale symétriquement sur les deux côtés.

2. Implant en particulier selon la revendication 1, **caractérisé en ce qu'**il est fabriqué en titane, en alliage de titane ou en nitinol.

3. Implant selon l'une quelconque des revendications 1 ou 2, **caractérisé en ce qu'**il présente des perforations (42).

4. Implant selon la revendication 3, **caractérisé en ce que** les perforations (42) sont prévues sur les bandes latérales (43, 44) et sur la partie centrale de la découpe (41) se trouvant entre elles.

5. Implant selon la revendication 3 ou 4, **caractérisé en ce que** les perforations (42) se présentent sous la forme de trous circulaires ou de trous oblongs.

6. Implant selon l'une quelconque des revendications 1 à 5, **caractérisé en ce qu'**il présente un revêtement toléré par le corps.

7. Implant selon l'une quelconque des revendications 1 à 6, **caractérisé en ce qu'**il est fabriqué au moyen d'une technique laser.
